# EUROPEAN PATENT SPECIFICATION

(11) **EP 1 513 565 B1**
(45) Date of publication and mention of the grant of the patent: **25.01.2012**
(21) Application number: 03739206.5
(22) Date of filing: 19.06.2003
(51) Int. Cl.: A61L 27/34, A61L 27/56, A61L 31/16, A61L 31/10, A61L 29/08, A61L 29/16

(54) **IMPLANTABLE OR INSERTABLE MEDICAL DEVICES FOR CONTROLLED DELIVERY OF A THERAPEUTIC AGENT**
IMPLANTIERBARE ODER EINSETZBARE MEDIZINISCHE VORRICHTUNG FÜR DIE KONTROLLIERTE ABGABE EINES THERAPEUTISCHEN MITTELS
DISPOSITIFS ELECTRONIQUES IMPLANTABLES OU INSERABLES ASSURANT LA LIBERATION REGULEE D'UN AGENT THERAPEUTIQUE

(30) Priority: 19.06.2002 US 174286
(43) Date of publication of application: 16.03.2005
(73) Proprietor: Boston Scientific Limited, St. Michael, Barbados, West Indies (BB)
(72) Inventor: SCHWARZ, Marlene, C., Auburndale, MA 02446 (US); RICHARD, Robert, E., Wrentham, MA 02093 (US)
(74) Representative: Vossius & Partner
(86) International application number: PCT/US2003/019309
(87) International publication number: WO 2004/000381

(56) References cited:
- EP-A- 0 328 421
- WO-A-00/41647
- WO-A-00/62830
- WO-A-02/47731
- WO-A-84/01721
- WO-A-86/02006
- WO-A-03/035135

## Description

### FIELD OF THE INVENTION

The present invention relates to implantable or insertable medical devices for controlled delivery of one or more therapeutic agents.

### BACKGROUND OF THE INVENTION

Numerous medical devices have been developed for the delivery of therapeutic agents to the body.

In accordance with some delivery strategies, a therapeutic agent is provided (a) within a polymeric carrier layer and/or (b) beneath a polymeric barrier layer that is associated with an implantable or insertable medical device. Once the medical device is placed at the desired location within a patient, the therapeutic agent is released from the medical device at a rate that is dependent upon the nature of the polymeric carrier and/or barrier layer.

The desired release profile for the therapeutic agent is dependent upon the particular treatment at hand, including the specific condition being treated, the specific therapeutic agent selected, the specific site of administration, and so forth. As a result, there is a continuing need for polymeric layers, including polymeric barrier layers and carrier layers, which are able to provide a broad range of therapeutic agent release rates. Various medical devices are known from WO 00/41647 A, WO 86/02006 A, WO 84/01721 A, EP-A-0328421, WO 00/62830 A and WO 03/035135 A.

### SUMMARY OF THE INVENTION

According to a first aspect of the present invention, a therapeutic-agent-releasing medical device according to claim 1 is provided.

In preferred embodiments, the first and second polymers are selected such that at least one polymer chain in the second polymer is compatible with at least one polymer chain in the first polymer. Two polymer chains are said to be compatible with one another when the phases that correspond to these chains exhibit at least some degree of interfacial mixing, up to and including complete miscibility between the phases.

In some embodiments, the release layer is a carrier layer that comprises the therapeutic agent(s). In other embodiments, the release layer is a barrier layer disposed over a therapeutic-agent-containing region, which comprises the therapeutic agent.

Preferred medical devices include catheters, guide wires, balloons, filters, stents, stent grafts, vascular grafts, vascular patches, shunts, and intraluminal paving systems. The device can be adapted, for example, for implantation or insertion into the coronary vasculature, peripheral vascular system, esophagus, trachea, colon, biliary tract, urinary tract, prostate or brain.

Beneficial therapeutic agents for the practice of the present invention include anti-thrombotic agents, anti-proliferative agents, anti-inflammatory agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, and agents that interfere with endogenous vasoactive mechanisms.

According to another aspect of the present invention, a method according to claim 4 is provided. Solvent spraying is a preferred technique for applying the above solution.

In some embodiments (for example, where a carrier layer is being formed), the solution further comprises the therapeutic agent. In other embodiments (for example, where a barrier layer is being formed), the solution is applied over a therapeutic-agent-containing region that comprises the therapeutic agent.

One advantage of the present invention is that implantable or insertable medical devices are provided, which are able to provide therapeutic agent release over a wide variety of time frames.

Another advantage of the present invention is that effective strategies are provided for controlling, or "tuning," the release profile of a therapeutic agent from an implantable or insertable medical device.

These and other embodiments and advantages of the present invention will become immediately apparent to those of ordinary skill in the art upon review of the Detailed Description and Claims to follow.

### BRIEF DESCRIPTION OF THE DRAWINGS

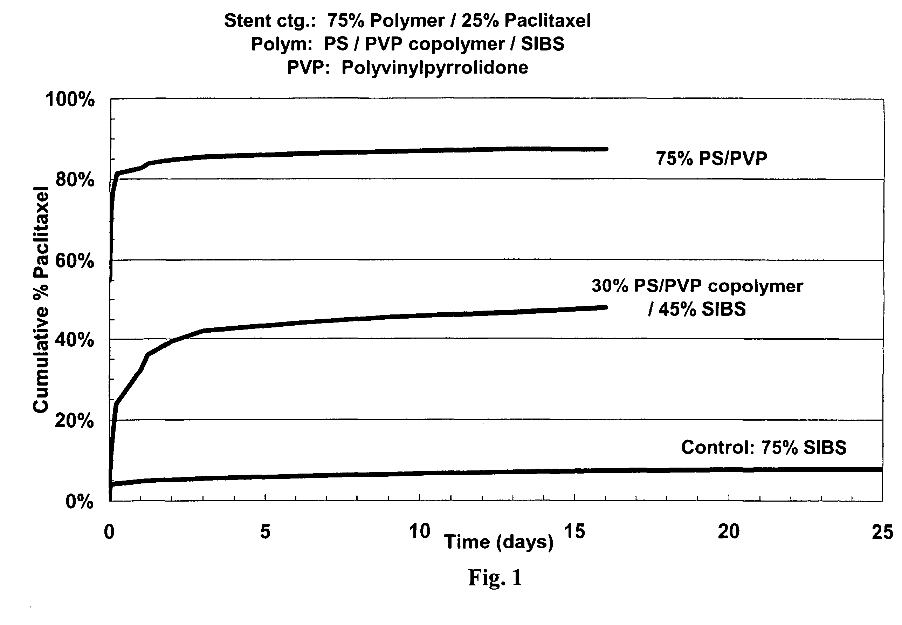

Fig. 1 illustrates cumulative release of paclitaxel as a function of time for carrier layers containing (a) polystyrene-polyisobutylene-polystyrene triblock copolymer, (b) polystyrene-polyvinylpyrrolidone diblock copolymer, and (c) a blend of polystyrene-polyisobutylene-polystyrene triblock copolymer with polystyrene-polyvinylpyrrolidone diblock copolymer, in accordance with an embodiment of the present invention.

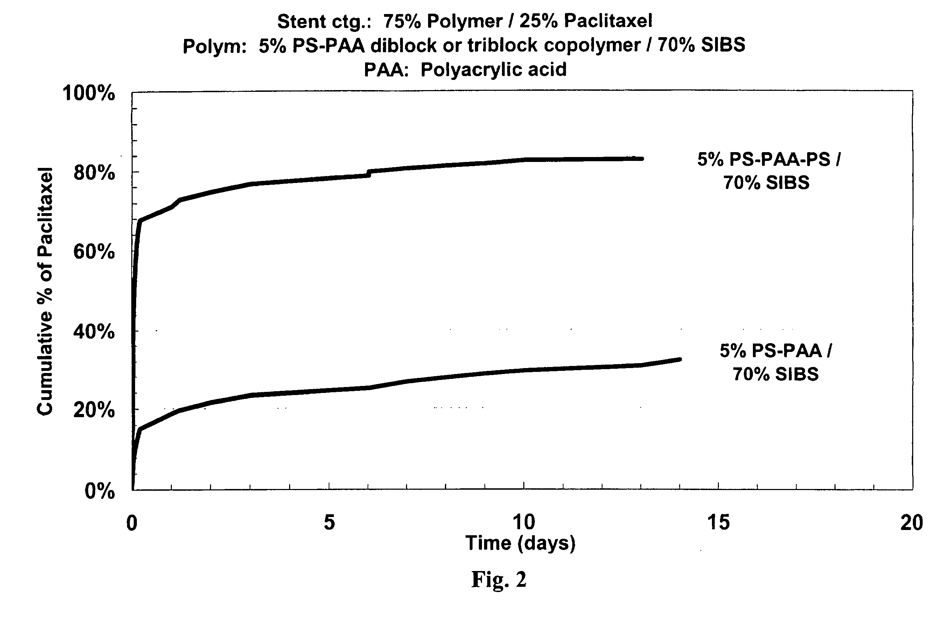

Fig. 2 illustrates cumulative release of paclitaxel as a function of time for carrier layers containing (a) a blend of polystyrene-polyisobutylene-polystyrene triblock copolymer and polystyrene-polyacrylic acid-polystyrene triblock copolymer, and (b) a blend of polystyrene-polyisobutylene-polystyrene block copolymer and polystyrene-polyacrylic acid diblock copolymer.

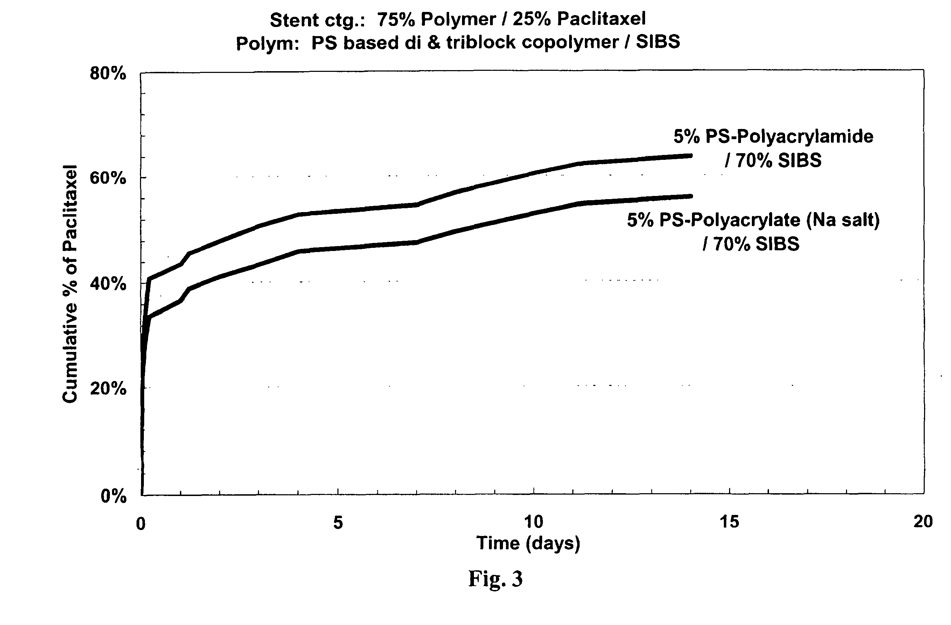

Fig. 3 illustrates cumulative release of paclitaxel as a function of time for carrier layers containing (a) a blend of polystyrene-polyisobutylene-polystyrene triblock copolymer and polystyrene-polyacrylamide diblock copolymer, and (b) a blend of polystyrene-polyisobutylene-polystyrene triblock copolymer and polystyrene-polyacrylate sodium salt diblock copolymer.

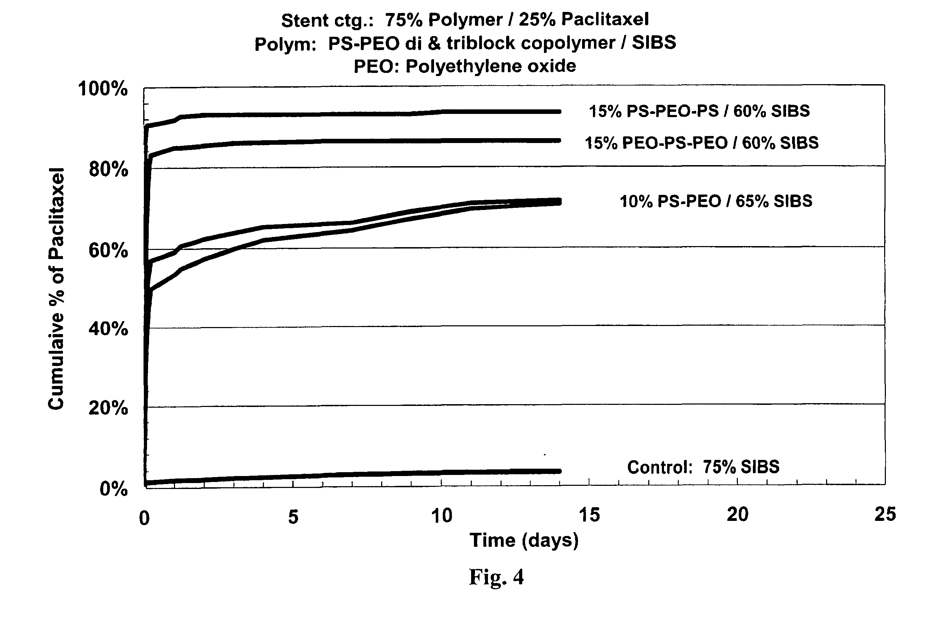

Fig. 4 illustrates cumulative release of paclitaxel as a function of time for carrier layers containing (a) a blend ofpolystyrene-polyisobutylene-polystyrene triblock copolymer and polystyrene-polyethylene oxide-polystyrene triblock copolymer, (b) a blend of polystyrene-polyisobutylene-polystyrene triblock copolymer and polyethylene oxide-polystyrene-polyethylene oxide triblock copolymer, and (c) blends of polystyrene-polyisobutylene-polystyrene triblock copolymer with two polystyrene-polyethylene oxide diblock copolymers.

### DETAILED DESCRIPTION OF THE INVENTION

The present invention relates to implantable or insertable medical device which provide for the release of one or more therapeutic agents, and methods for making such devices. Herein disclosed are further methods for modulating the rate at which therapeutic agent is released from such devices.

By "release layer" is meant a layer that regulates the rate of release of at least one therapeutic agent. Two preferred release layers for use in accordance with the present invention are carrier layers and barrier layers.

By "carrier layer" is meant a layer which contains at least one therapeutic agent and from which the therapeutic agent is released.

By "barrier layer" is meant a layer which is disposed between a source of therapeutic agent and a site of intended release and which controls the rate at which the therapeutic agent is released.
The first polymer will be a block copolymer or a graft copolymer, in which case multiple phase domains are typically formed.

First polymers for use in the present invention are block or graft copolymers comprising at least two polymer chains X and Y. The X polymer chains are based upon isobutylene. The Y polymer chains are polymers of vinyl aromatics, such as chains made from monomers of styrene 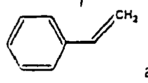 and/or styrene derivatives (e.g., α-methylstyrene, ring-alkylated styrenes or ring-halogenated styrenes or other substituted styrenes where one or more substituents are present on the aromatic ring), collectively referred to herein as "styrenic blocks" or "styrenic chains".

Examples include diblock copolymers, triblock copolymers, star copolymers, and branched block copolymers, for example, dendritic block copolymers (e.g., arborescent block copolymers) wherein at least one of the X and Y chains is branched, and preferably wherein the X chains are branched and capped by the Y chains.

In some particularly preferred embodiments of the present invention the first polymer is selected from (a) polystyrene-polyisobutylene-polystyrene triblock copolymers (these polymers typically form two phase domains when the first polymer is in a pure solid-state form-a phase domain corresponding to the polystyrene polymer chains and a phase domain corresponding to the polyisobutylene polymer chain), which, along with other polymers appropriate for the practice of the present invention, are described, for example, in U.S. Patent No. 5,741,331, U.S. Patent No. 4,946,899 and U.S. Serial No. 09/734,639, or (b) arborescent polyisobutylene-polystyrene block copolymers such as those described in Kwon et al., "Arborescent Polyisobutylene-Polystyrene Block Copolymers-a New Class of Thermoplastic Elastomers," Polymer Preprints, 2002,43(1), 266,

The second polymer is a block copolymer or a graft copolymer containing two or more polymer chains, which give rise to two or more phase domains when the first polymer is in a pure solid-state form. As previously noted, the second polymer is preferably selected such that least one polymer chain in the second polymer is compatible with at least one polymer chain in the first polymer. More preferably, the same monomer or monomers are used to form the compatible polymer chains in both the first and second polymers.

For example, continuing with the specific example above in which the first polymer is a polystyrene-polyisobutylene-polystyrene triblock copolymer, it is preferred that the second polymer contain (a) one or more chains of polyisobutylene or (b) one or more chains of polystyrene.

Examples of second polymers containing one or more chains of polyisobutylene include block copolymers and graft copolymers comprising (i) one or more polyisobutylene chains and (ii) one or more chains selected from polyethylene oxide; polyvinylpyrrolidone; polyacrylamide; polydimethylacrylamide; polyacrylic acid and salts thereof; polymethacrylic acid and salts thereof; poly(maleic anhydride) and partial esters, free acids and acid salts thereof; polyvinyl alcohol and fully and partially hydrolyzed derivatives thereof; and poly(vinyl pyridine).

Examples of second polymers containing one or more chains of polystyrene include block copolymers and graft copolymers comprising (i) one or more chains of polystyrene and (ii) one or more chains selected from polyethylene oxide; polyvinylpyrrolidone; polyacrylamide; polydimethylacrylamide; polyacrylic acid and salts thereof; polymethacrylic acid and salts thereof; poly(maleic anhydride) and partial esters, free acids and acid salts thereof; polyvinyl alcohol and fully and partially hydrolyzed derivatives thereof; and poly(vinyl pyridine).

The table below lists several specific copolymers for use in connection with the present invention:

| **Polymer** | **Mol. Wt.** | **Source** |
|---|---|---|
| PIB-PEO diblock copolymer | 5kDa - 15kDa | PSI |
| PS-PEO diblock copolymer | 11kDa - 41.9kDa | PSI |
| PS-PEO diblock copolymer | 58.6kDa - 71kDa | PSI |
| PS-PEO-PS triblock copolymer | 9.5kDa - 48kDa - 9.5kDa | PSI |
| PEO-PS-PEO triblock copolymer | 6.3kDa - 1.7kDa - 6.3kDa | PSI |
| PVP-PS diblock copolymer | 100kDa (overall) | ISP |
| PS-P(NaAc) diblock copolymer | 3.5kDa - 34.5kDa | PSI |
| PS-PA diblock copolymer | 16.5kDa - 4.4kDa | PSI |
| PS-PAA-PS triblock copolymer | 2.5kDa - 22.8kDa - 2.5kDa | PSI |
| PS-PAA diblock copolymer | 4.5kDa - 22.8kDa | PSI |
| PS-P(NaMeAc) diblock copolymer | | PSI |
| PS-PDM diblock copolymer | | PSI |
| PS grafted PEO copolymer | | PSI |
| PS grafted PA copolymer | | PSI |
| PS-PEO diblock copolymer | 3.8kDa - 6.5kDa | PSI |
| PS-PEO diblock copolymer | 4.6kDa - 12.2kDa | PSI |
| | | |

| | | |
|---|---|---|
| Abbreviations: PIB =polyisobutylene PS = polystyrene PEO = polyethylene oxide PVP = polyvinylpyrrolidone P(NaAc) = polyacrylate sodium salt P(NaMeAc) = polymethacrylate sodium salt PDM = polydimethylacrylamide PA = polyacrylamide PAA = polyacrylic acid PSI = Polymer Source Inc., Dorval, Quebec, Canada ISP = International Specialty Products, Wayne, NJ, USA | | |

The hydrophobic/hydrophilic balance of a release layer comprising a first polymer like that described above can be modulated (or "tuned") by adding a second polymer like that described above. This change in the hydrophobic/hydrophilic balance of the release layer is accompanied by a change in the rate of release of therapeutic agent

As an example, a release layer, which comprises a first polymer consisting one or more hydrophilic polymer chains, can be rendered more hydrophobic by adding a second polymer, which comprises (a) one or more hydrophilic polymer chains that are compatible with the hydrophilic polymer chains within the first polymer and (b) one or more hydrophobic polymer chains.

As used herein, a "hydrophilic polymer chain" is one that is more compatible with water and/or more polar in nature compared to other polymer chains while a "hydrophobic polymer chain" is one that is less compatible with water and/or less polar in nature compared to other polymer chains.

Conversely, as another specific example, a release layer, which comprises a first polymer that consists of one or more hydrophobic polymer chains, can be rendered more hydrophilic by adding a second polymer that comprises (a) one or more hydrophobic polymer chains that are compatible with the hydrophobic polymer chains within the first polymer and (b) one or more hydrophilic polymer chains.

For instance, release layers comprising a first polymer that contains polyisobutylene and polystyrene chains, both of which are hydrophobic (see, e.g., the polystyrene-polyisobutylene-polystyrene triblock copolymer of the Examples), can be rendered more hydrophilic by adding block copolymers which comprise (a) one or more polystyrene chains (which are hydrophobic and compatible with the polystyrene chains within the triblock copolymer) and (b) one or more hydrophilic polymer chains, such as the following: (i) polyvinylpyrrolidone (see, e.g., the polystyrene-polyvinylpyrrolidone diblock copolymer of Example 1), (ii) polyacrylic acid and its salts (see, e.g., the polystyrene-polyacrylic acid diblock copolymer of Example 2, the polystyrene-polyacrylic acid-polystyrene triblock copolymer of Example 2, and the polystyrene-sodium polyacrylate diblock copolymer of Example, 3), (iii) polyacrylamide (see, e.g., the polystyrene-polyacrylamide diblock copolymer of Example 3), and (iv) polyethylene oxide (see, e.g., the polystyrene-polyethylene oxide-polystyrene triblock copolymer of Example 4, the polyethylene oxide-polystyrene-polyethylene oxide triblock copolymer of Example 4, and the polystyrene-polyethylene oxide diblock copolymer of Example 4).

These specific embodiments are advantageous for several reasons including the following: (1) Because the first polymer contains only hydrophobic polymer chains, and because the second polymer contains at least one hydrophilic polymer chain, the release layer is rendered more hydrophilic upon the addition of the second polymer. This typically results in a modulation of the drug release rate from the release layer. (2) Also, hydrophilic polymer chains, such as polyethylene oxide chains, are commonly prone to dissolution from release layers upon implantation/insertion, due to their compatibility with the surrounding aqueous environment. However, by attaching the hydrophilic polymer chain to a hydrophobic polymer chain (e.g., by attaching polyethylene oxide to polystyrene), the hydrophilic polymer chain is "anchored" by the hydrophobic polymer chain, rendering the hydrophilic polymer chain less prone to dissolution into a surrounding aqueous environment.

Hence, by following the above and analogous principles, the hydrophilic/hydrophobic balance of a release layer containing a first polymer like that described above can be altered by adding a second polymer like that described above. Whether or not the release layer will be rendered more hydrophilic or more hydrophobic upon the addition of the second polymer will depend upon on the composition and length of the polymer chains that are present within the first and second polymers.

The release rate can also be modulated by varying the composition and/or length of the polymer chains within the second polymer. As a specific example, a release layer comprising a polystyrene-polyisobutylene-polystyrene triblock copolymer as a first polymer and a polystyrene-polyethylene oxide block copolymer as a second polymer can be rendered more hydrophilic by either (a) substituting a more hydrophilic polymer chain for the polyethylene oxide chain within the polystyrene-polyethylene oxide block copolymer, or (b) increasing the length of the polyethylene oxide chain within the polystyrene-polyethylene oxide block copolymer.

The hydrophilic/hydrophobic balance of the release layer can be changed to modulate the release rate. However, other characteristics of the release layer besides hydrophilicity (or hydrophobicity, if viewed from the opposite perspective), for example, the overall polarity or ionic character of the release layer, may be modified by changing the nature of the second polymer (for example, by changing the length and/or composition of the polymer chains within the second polymer).

Preferred implantable or insertable medical devices for use in connection with the release layers of the present invention include catheters (for example, renal or vascular catheters such as balloon catheters), guide wires, balloons, filters (e.g., vena cava filters), stents (including coronary vascular stents, cerebral, urethral, ureteral, biliary, tracheal, gastrointestinal and esophageal stents), stent grafts, cerebral aneurysm filler coils (including GDC--Guglilmi detachable coils--and metal coils), vascular grafts, myocardial plugs, patches, pacemakers and pacemaker leads, heart valves, biopsy devices, or any coated substrate (which can comprise, for example, glass, metal, polymer, ceramic and combinations thereof) that is implanted or inserted into the body, either for procedural use or as an implant, and from which therapeutic agent is released.

The medical devices contemplated for use in connection with the present invention include drug delivery medical devices that are used for either systemic treatment or for the localized treatment of any mammalian tissue or organ. Non-limiting examples are tumors; organs including but not limited to the heart, coronary and peripheral vascular system (referred to overall as "the vasculature"), lungs, trachea, esophagus, brain, liver, kidney, bladder, urethra and ureters, eye, intestines, stomach, pancreas, ovary, and prostate; skeletal muscle; smooth muscle; breast; cartilage; and bone.

One particularly preferred medical device for use in connection with the present invention is a vascular stent, which delivers therapeutic agent into the vasculature, for example, in the treatment of restenosis. As used herein, "treatment" refers to the prevention of a disease or condition, the reduction or elimination of symptoms associated with a disease or condition, or the substantial or complete elimination of a disease or condition. Preferred subjects are mammalian subjects and more preferably human subjects.

"Therapeutic agents", "pharmaceutically active agents", "pharmaceutically active materials", "drugs" and other related terms may be used interchangeably herein and include genetic therapeutic agents, non-genetic therapeutic agents and cells. Therapeutic agents may be used singly or in combination.

Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; and (o)agents that interfere with endogenous vascoactive mechanisms.

Exemplary genetic therapeutic agents for use in connection with the present invention include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, Bump-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include (a) plasmids, (b) viral vectors such as adenovirus, adenoassociated virus and lentivirus, and (c) non-viral vectors such as lipids, liposomes and cationic lipids.

Cells for use in connection with the present invention include cells of human origin (autologous or allogeneic), including stem cells, or from an animal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (l) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM- and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs(6-mercaptopurine), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925.

A wide range of therapeutic agent loadings can be used in connection with the medical devices of the present invention, with the amount of loading being readily determined by those of ordinary skill in the art and ultimately depending, for example, upon the condition to be treated, the nature of the therapeutic agent itself, the means by which the therapeutic agent is administered to the intended subject, and so forth.

In general, the release layers of the present invention are formed using any appropriate technique known in the art. Solvent-based techniques are preferred, in which the above-described first and second polymers are dissolved or dispersed in a solvent system prior to layer formation.

Where solvent-based techniques are used, the solvent system that is selected will contain one or more solvent species. The solvent system preferably is a good solvent for the polymers and, where included, for the therapeutic agent as well. The particular solvent species that make up the solvent system may also be selected based on other characteristics including drying rate and surface tension.

Solvent species that can be used in connection with the present invention include any combination of one or more of the following: (a) water, (b) alkanes such as ethane, hexane, octane, cyclohexane, heptane, isohexane, butane, pentane, isopentane, 2,2,4-trimethlypentane, nonane, decane, dodecane, hexadecane, eicosane, methylcyclohexane, cis-decahydronaphthalene and trans-decahydronaphthalene, (c) aromatic species such as benzene, toluene, xylene(s), naphthalene, styrene, ethylbenzene, 1-methylnaphthalene, 1,3,5-trimethylbenzene, tetrahydronaphthalene, diphenyl and 1,4-diethylbenzene, (d) halohydrocarbons including (i) chlorohyhdrocarbons such as chloroform, methyl chloride, dichloromethane, 1,1-dichloroethylene, ethylene dichloride, ethylidene chloride, propyl chloride, cyclohexyl chloride, 1,1,1-trichloroethane, perchloroethylene, trichloroethylene, butyl chloride, carbon tetrachloride, tetrachloroethylene, chlorobenzene, o-dichlorobenzene, benzyl chloride, trichlorobiphenyl, methylcyclohexane, 1,1,2,2-tetrachloroethane (ii) fluorinated halogenated species such as chlorodiflouoromethane, dichlorofluoromethane, dichlorodifluoromethane, trichlorofluoromethane, 1,2-dichlorotetrafluoroethane, 1,1,2-trichlorotrifluoroethane, perfluor(methylcyclohexane), perfluor(dimethylcyclohexane) and (iii) other halohydrocarbons such as ethyl bromide, ethylidene bromide, ethylene dibromide, tribromomethane, bromotrifluoromethane, 1,1,2,2-tetrabromoethane, bromobenzene, bromochloromethane, 1-bromonaphthalene, methyl iodide, methylene diiodide (e) acid aldehydes/anhydrides such as acetaldehyde, furfural, butyraldehyde, benzaldehyde, acetyl chloride, succinic anhydride and acetic anhydride, (f) alcohols including (i) phenols such as phenol, 1,3-benzenediol, m-cresol, o-methoxyphenol, methyl salicylate and nonylphenol, (ii) polyhydric alcohols such as ethylene glycol, glycerol, propylene glycol, 1,3-butanediol, diethylene glycol, triethylene glycol, hexylene glycol and dipropylene glycol, and (iii) other alcohols such as methanol, ethanol, ethylene cyanohydrin, allyl alcohol, 1-propanol, 2-propanol, 3-chloropropanol, furfuryl alcohol, 1-butanol, 2-butanol, benzyl alcohol, isobutanol, cyclohexanol, 1-pentanol, 2-ethyl-1-butanol, diacetone alcohol, 1,3-dimethyl-1-butanol, ethyl lactate, butyl lactate, ethylene glycol monomethyl ether, ethylene glycol monoethyl ether, diethylene glycol monomethyl ether, diethylene glycol monoethyl ether, ethylene glycol monobutyl ether, 2-ethyl-1-hexanol, 1-octanol, 2-octanol, diethylene glycol monobutyl ether, 1-decanol, 1-tridecyl alcohol, nonyl-phenoxy ethanol, oleyl alcohol, triethylene glycol mono-oleyl ether, (g) ethers such as, epichlorohydrin, furan, 1,4-dioxane, dimethoxymethane, diethyl ether, bis-(2-chloroethyl) ether, anisole, di-(2-methoxyethyl) ether, dibenzyl ether, di-(2-chloroisopropyl) ether, bis-(m-phenoxyphenol) ether, dimethyl ether and tetrahydrofuran, (h) ketones, such as acetone, cylohexanone, isophorone, diethyl ketone, mesityl oxide, acetophenone, methyl ethyl ketone, methyl isoamyl ketone, methyl isobutyl ketone, and methyl propyl ketone, (i) acids such as formic acid, acetic acid, benzoic acid, butyric acid, octanoic acid, oleic acid, stearic acid, (j) esters/acetates such as ethylene carbonate, butyrolactone, propylene-1,2-carbonate, ethyl chloroformate, ethyl acetate, trimethyl phosphate, diethyl carbonate, diethyl sulfate, ethyl formate, methyl acetate, n-butyl acetate, isobutyl acetate, t-butyl acetate, 2-ethoxyethyl acetate, isoamyl acetate, dimethyl phthalate, ethyl cinnamate, triethyl phosphate, diethyl phosphate, butyl benzyl phthalate, dibutyl phthalate, diethyl phthalate, tricrysyl phosphate, tributyl phosphate, dibutyl sebacate, methyl oleate, dioctyl phthalate, dibutyl stearate isopropyl acetate, isobutyl isobutyrate, n-propyl acetate and n-butyl propionate, (k) nitrogen compounds such as acetonitrile, acrylonitrile, propionitrile, butyronitrile, nitromethane, nitroethane, 2-nitropropane, nitrobenzene, ethanolamine, ethylenediamine, 1,1-dimethylhydrazine, 2-pyrrolidone, pyridine, propylamine, morpholine, analine, n-methyl-2-pyrrolidone, butylamine, diethylamine, cyclohexylamine, quinoline, dipropylamine, formamide, n,n-dimethylformamide, n,n-dimethylacetamide, tetramethylurea, hexamethyl phosphoramide, diethylenetriamine, triethylamine and triethanolamine, and (l) sulfur compounds such as carbon disulfide, dimethylsulfoxide, ethanethiol, dimethyl sulfone and diethyl sulfide.

Preferred solvent-based techniques include, but are not limited to, solvent casting techniques, spin coating techniques, web coating techniques, solvent spraying techniques, dipping techniques, techniques involving coating via mechanical suspension, including air suspension, ink jet techniques, electrostatic techniques, and combinations of these processes. Typically, a solution containing solvent and polymers (and, in some cases, a therapeutic agent) is applied to a substrate to form a release layer (e.g., a carrier layer or barrier layer). The substrate is typically all or a portion of an implantable or insertable medical device, to which the release layer is applied.

Where appropriate, techniques such as those listed above can be repeated or combined to build up a release layer to a desired thickness. The thickness of the release layer can be varied in other ways as well. For example, in one preferred process, solvent spraying, coating thickness can be increased by modification of coating process parameters, including increasing spray flow rate, slowing the movement between the substrate to be coated and the spray nozzle, providing repeated passes and so forth.

In the case where a carrier layer is being established, a therapeutic agent can be included in the above-described polymer solution if desired, and hence co-established with the carrier layer. In other embodiments, on the other hand, the therapeutic agent can be dissolved or dispersed within a solvent, and the resulting solution contacted with a previously formed carrier layer, for example, using one or more of the application techniques described above (e.g., dipping, spraying, etc.).

Barrier layers, on the other hand, are formed over a therapeutic-agent-containing region. In some embodiments, however, the therapeutic-agent-containing region comprises one or more polymers, which can be selected, for example, from the polymers listed above. As such, the therapeutic-agent-containing region can also be established using solvent-based techniques (e.g., dipping, spraying, etc.) such as those discussed above. In other embodiments, the therapeutic-agent-containing region beneath the barrier layer is established without an associated polymer. For example, the therapeutic agent can simply be dissolved or dispersed in a liquid, and the resulting solution/dispersion contacted with a substrate, for instance, using one or more of the above-described application techniques.

Where the release layer is formed using a solvent based technique, it is preferably dried after application to remove the solvents. The release layer typically further conforms to the underlying surface during the drying process.

The invention is further described with reference to the following non-limiting Examples.

### EXAMPLE 1

Solutions are provided that contain 99 wt% chloroform, 0.25 wt% paclitaxel and 0.75 wt% of a polymer composition or blend.

One solution is prepared by mixing 0.75 wt% of the block copolymer polystyrene-polyisobutylene-polystyrene block copolymer (SIBS) with the solvent and paclitaxel. The SIBS copolymer is synthesized using known techniques such as those described in U.S. Patent No. 5,741,331, U.S. Patent No. 4,946,899 and U.S. Serial No. 09/734,639.

A second solution is prepared by mixing 0.75 wt% of the block copolymer polystyrene-polyvinylpyrrolidone (PS/PVP) with the solvent and paclitaxel. The PS/PVP copolymer is available from International Specialty Products Corporation (ISP) as Agrimer™ ST.

A third solution is prepared by blending 0.30% of the PS/PVP copolymer and 0.45% of the SIBS copolymer with the solvent and paclitaxel.

All solutions are prepared by (1) mixing the paclitaxel and a small amount of the chloroform, (2) adding the polymer or copolymers, (3) adding the remaining chloroform, (4) thoroughly mixing (e.g., overnight), and (5) filtering.

The solution is then placed in a syringe pump and fed to a spray nozzle. A stent is mounted onto a holding device parallel to the nozzle and, if desired, rotated to ensure uniform coverage. Depending on the spray equipment used, either the component or spray nozzle can be moved while spraying such that the nozzle moves along the component while spraying for one or more passes. After a carrier coating is formed in this fashion, the stent is dried, for example, by placing it in a preheated oven for 30 minutes at 65°C, followed by 3 hours at 70°C.

Three stents are formed in this manner for each of the various solutions.

Paclitaxel release is then measured as a function of time in PBS containing 0.5 wt% Tween® 20 (polyoxyethylene(20) sorbitan monolaurate) available from Sigma-Aldrich. The results, presented as the cumulative release of paclitaxel as a function of time, are graphically illustrated in Fig. 1.

These results indicate that the release rate of a therapeutic agent from a polymeric carrier layer can be modulated by changing the ratio of the hydrophilic and hydrophobic polymeric components by blending a hydrophobic polymeric drug carrier with a block copolymer containing at least one hydrophilic polymer chain and at least one hydrophilic polymer chain.

### EXAMPLE 2

A series of solutions are prepared in a procedure similar to the procedure used in Example 1. All solutions contain the following: 25 wt% tetrahydrofuran (THF), 74 wt% toluene, 0.25 wt% paclitaxel and 0.75 wt% of a polymer composition or blend.

Solutions are made containing the following polymeric constituents: (a) 0.05 wt% of a polystyrene-polyacrylic acid-polystyrene triblock copolymer (PS-PAA-PS) with block lengths of 3.3k-13.5k-3.3k, respectively, and 0.70 wt% SIBS, and (b) 0.05 wt% of a polystyrene-polyacrylic acid diblock copolymer (PS-PAA) with block lengths of 4.3k-19.5k, respectively, and 0.70 wt% SIBS. The PS-PAA-PS and PS-PAA copolymers are available from the Polymer Source, Inc.

All solutions are prepared by (1) mixing the paclitaxel and THF, (2) adding the polymer or copolymers, (3) adding the toluene, (4) thoroughly mixing (e.g., overnight), and (5) filtering.

The solutions are applied to stents and dried according to the procedures of Example 1. Three stents are coated using each of the above solutions. The cumulative release of paclitaxel as a function of time is then measured as in Example 1. The results are graphically illustrated in Fig. 2.

These results indicate that the release rate of a therapeutic agent from a polymeric carrier layer can be modulated by changing the ratio of the hydrophilic and hydrophobic polymeric components by blending a hydrophobic polymeric drug carrier with a block copolymer that contains at least one hydrophilic polymer chain and at least one hydrophobic polymer chain.

### EXAMPLE 3

A series of solutions are prepared in a procedure similar to the procedure used in Example 1. The solutions contain the following: dimethyl acetamide (DMAc), toluene, 0.25 wt% paclitaxel and 0.75 wt% of a polymer composition or blend.

The solutions are made containing the following polymeric constituents: (a) 0.05 wt% of a polystyrene-polyacrylamide diblock copolymer (PS-Polyacrylamide) with block lengths of 16.5k-4.4k, respectively, and 0.70 wt% SIBS, (b) 0.05 wt% of a polystyrene-polyacrylate sodium salt diblock copolymer (PS-Polyacrylate (Na salt)) with block lengths of 3.5k-34.5k, respectively, and 0.70 wt% SIBS. The PS-Polyacrylamide and PS-Polyacrylate (Na salt) copolymers are available from the Polymer Source, Inc.

All solutions are prepared by (1) mixing the paclitaxel and 15-17 wt% DMAc, (2) adding the polymers or copolymers, (3) adding 84-82 wt% toluene as required to make a solution with 1.0 wt% total solids, (4) thoroughly mixing (e.g., overnight), and (5) filtering

The solutions are applied to stents and dried according to the procedures of Example 1. Three stents are coated using each of the above solutions. The cumulative release of paclitaxel as a function of time is then measured as in Example 1. The results are graphically illustrated in Fig. 3.

These results indicate that the release rate of a therapeutic agent from a polymeric carrier layer can be modulated by changing the ratio of the hydrophilic and hydrophobic polymeric components by blending a hydrophobic polymeric drug carrier with a block copolymer that contains at least one hydrophilic polymer chain and at least one hydrophobic polymer chain.

### EXAMPLE 4

A series of solutions are prepared in a procedure similar to the procedure used in Example 1. All solutions contain the following: 99% chloroform, 0.25 wt% paclitaxel and 0.75 wt% of a polymer composition or blend.

The control solution is prepared by mixing 0.75 wt% the SIBS copolymer (see Example 1) with the solvent and paclitaxel.

Test solutions are made containing the following polymeric constituents: (a) 0.15 wt% Polystyrene-co-polyethylene oxide-co-polystyrene triblock copolymer (PS-PEO-PS) with block lengths of 9.5k-49k-9.5k, respectively, and 0.60 wt% SIBS, (b) 0.15 wt% Polyethylene oxide-co-polystyrene-co-polystyrene triblock copolymer (PEO-PS-PEO) with block lengths of 6.3k-1.7k-6.3k, respectively, and 0.60 wt% SIBS, (c) 0.10 wt% Polystyrene-co-polyethylene oxide diblock copolymer (PS-PEO) with block lengths of 58.6k-71k, and 11k-41.9k, respectively, and 0.65 wt% SIBS. The polystyrene and polyethylene oxide diblock and triblock copolymers are available from the Polymer Source, Inc.

The solutions are applied to stents and dried according to the procedures of Example 1. Three stents are coated using each of the above solutions. The cumulative release of paclitaxel as a function of time is then measured as in Example 1. The results are graphically illustrated in Fig. 4.

These results indicate that the release rate of a therapeutic agent from a carrier layer comprising a hydrophobic polymeric carrier can be modulated by the addition of block copolymers with hydrophilic and hydrophobic polymer chains. The results also show that the release rate is a function of the polymer structure (i.e. triblock copolymer compared to a diblock copolymer), and may be altered by changing the relative amount of the hydrophilic and hydrophobic polymer chains of the block copolymer.

## Claims

1. A therapeutic-agent-releasing medical device comprising:
(a) an implantable or insertable medical device;
(b) a release layer disposed over at least a portion of the implantable or insertable medical device, said release layer comprising
(A) a first polymer which is a block or graft copolymer comprising (i) one or more polyisobutylene polymer chains and (ii) one or more styrenic polymer chains; and
(B) a second polymer is a block or graft copolymer comprising (i) one or more polyisobutylene polymer chains and (ii) one or more chains selected from polyethylene oxide; polyvinylpyrrolidone; polyacrylamide; polydimethylacrylamide; polyacrylic acid and salts thereof; polymethacrylic acid and salts thereof; poly(maleic anhydride) and partial esters, free acids and acid salts thereof; polyvinyl alcohol and fully and partially hydrolyzed derivatives thereof; and poly(vinyl pyridine),
or said second polymer (B) is a block or graft copolymer comprising (i) one or more styrenic polymer chains and (ii) one or more chains selected from polyethylene oxide; polyvinylpyrrolidone; polyacrylamide; polydimethylacrylamide; polyacrylic acid and salts thereof; polymethacrylic acid and salts thereof; poly(maleic anhydride) and partial esters, free acids and acid salts thereof; polyvinyl alcohol and fully and partially hydrolyzed derivatives thereof; and poly(vinyl pyridine); and (c) a therapeutic agent, wherein the phases that correspond to the polymer chains are miscible.

2. The medical device of claim 1,
wherein said release layer is a barrier layer disposed over a therapeutic-agent-containing region that comprises said therapeutic agent, or
wherein said release layer is a carrier layer comprising said therapeutic agent.

3. The medical device of claim 1,
wherein said implantable or insertable medical device is selected from a catheter, a guide wire, a balloon, a filter, a stent, a stent graft, a vascular graft, a vascular patch, a shunt, and an intraluminal paving system.

4. A method of forming the therapeutic-agent-releasing medical device of claim 1, comprising: (a) providing a solution comprising: one or more solvents, said first polymer (A) and said second polymer (B) ; (b) applying said solution to a surface of said implantable or insertable medical device; and (c) removing said solvents from said solution to form said release layer.

5. The method of claim 4, wherein said solution further comprises said therapeutic agent.

6. The method of claim 4, wherein said solution is applied over a therapeutic-agent-containing region that comprises said therapeutic agent.

7. The method of claim 4, wherein said solution is applied by a solvent spraying technique.

8. Use of a therapeutic-agent-releasing medical device of one of the claims 1 to 3 for the preparation of a device for the treatment of restenosis, or the treatment of diseases and/or tumors relating to organs comprising the colon, heart, coronary and peripheral vascular system, lungs, trachea, esophagus, brain, liver, kidney, bladder, urethra and ureters, eye, intestines, stomach, pancreas, ovary, or prostate, or the treatment of tumors relating to tissue comprising skeletal muscle, smooth muscle, breast, cartilage, or bone, wherein the device is prepared for implantation or insertion into said patient.

9. Use according to claim 8, wherein
said medical device is selected from a catheter,
a guide wire, a balloon, a filter, a stent, a stent graft, a vascular graft, a vascular patch, a shunt, and an intraluminal paving system, or
said medical device is prepared for insertion into the vasculature.

## Patentansprüche

1. Eine ein therapeutisches Mittel freisetzende medizinische Vorrichtung, umfassend:
(a) eine implantierbare oder einsetzbare medizinische Vorrichtung;
(b) eine Trennschicht, aufgebracht auf wenigstens einen Teil der implantierbaren oder einsetzbaren medizinischen Vorrichtung, wobei die Trennschicht umfasst
(A) ein erstes Polymer, welches ein Block- oder Pfropfcopolymer ist, umfassend (i) eine oder mehrere Polyisobutylen-Polymerkette(n) und (ii) eine oder mehrere styrolische Polymerkette(n); und
(B) ein zweites Polymer, welches ein Block- oder Pfropfcopolymer ist, umfassend (i) eine oder mehrere.Polyisobutylen-Polymerkette(n) und (ii) eine oder mehrere Kette(n) ausgewählt aus Polyethylenoxid; Polyvinylpyrrolidon; Polyacrylamid; Poly(dimethylacrylamid); Polyacrylsäure und Salze davon; Polymethacrylsäure und Salze davon; Poly(maleinsäureanhydrid) und partielle Ester, freie Säuren und Säuresalze davon; Polyvinylalkohol und vollständig und partiell hydrolysierte Derivate davon; und Poly(vinylpyridin),
oder das zweite Polymer (B) ist ein Block- oder Pfropfcopolymer, umfassend (i) eine oder mehrere styrolische Polymerkette(n) und (ii) eine oder mehrere Kette(n) ausgewählt aus Polyethylenoxid; Polyvinylpyrrolidon; Polyacrylamid; Poly(dimethylacrylamid); Polyacrylsäure und Salze davon; Polymethacrylsäure und Salze davon; Poly(maleinsäureanhydrid) und partielle Ester, freie Säuren und Säuresalze davon; Polyvinylalkohol und vollständig und partiell hydrolysierte Derivate davon; und Poly(vinylpyridin); und
(c) ein therapeutisches Mittel, wobei die Phasen, die den Polymerketten entsprechen, mischbar sind.

2. Die medizinische Vorrichtung gemäß Anspruch 1,
wobei die Trennschicht eine Sperrschicht ist, aufgebracht auf eine ein therapeutisches Mittel enthaltende Region, die das therapeutische Mittel umfasst, oder
wobei die Trennschicht eine Trägerschicht ist, umfassend das therapeutische Mittel.

3. Die medizinische Vorrichtung gemäß Anspruch 1,
wobei die implantierbare oder einsetzbare medizinische Vorrichtung aus einem Katheter, einem Führungsdraht, einem Ballon, einem Filter, einem Stent, einem Stentgraft, einem vaskulären Graft, einem vaskulären Patch, einem Shunt und einem intraluminalen Pflastersystem ausgewählt ist.

4. Ein Verfahren zur Herstellung der ein therapeutisches Mittel freisetzenden medizinischen Vorrichtung gemäß Anspruch 1, umfassend: (a) Bereitstellen einer Lösung umfassend: ein oder mehrere Lösungsmittel, das erste Polymer (A) und das zweite Polymer (B); (b) Aufbringen der Lösung auf eine Oberfläche der implantierbaren oder einsetzbaren medizinischen Vorrichtung; und (c) Entfernen der Lösungsmittel aus der Lösung, um die Trennschicht zu bilden.

5. Das Verfahren gemäß Anspruch 4, wobei die Lösung ferner das therapeutische Mittel umfasst.

6. Das Verfahren gemäß Anspruch 4, wobei die Lösung auf einer ein therapeutisches Mittel enthaltenden Region aufgebracht wird, die das therapeutische Mittel umfasst.

7. Das Verfahren gemäß Anspruch 4, wobei die Lösung durch eine -Lösungsmittelsprühtechnik aufgebracht wird.

8. Verwendung einer ein therapeutisches Mittel freisetzenden medizinischen Vorrichtung gemäß einem der Ansprüche 1 bis 3 zur Herstellung einer Vorrichtung zur Behandlung von Restenose oder zur Behandlung von Krankheiten und/oder Tumoren betreffend Organe umfassend das Kolon, das Herz, das koronare und das periphere Gefäßsystem, die Lungen, die Luftröhre, die Speiseröhre, das Gehirn, die Leber, die Niere, die Blase, die Harnröhre und den Harnleiter, das Auge, den Darm, den Magen, das Pankreas, die Eierstöcke oder die Prostata, oder zur Behandlung von Tumoren betreffend Gewebe umfassend die Skelettmuskeln, die glatten Muskeln, die Brust, die Knorpel oder die Knochen, wobei die Vorrichtung zur Implantation oder zum Einsetzen in den Patienten vorbereitet ist.

9. Verwendung gemäß Anspruch 8, wobei
die medizinische Vorrichtung aus einem Katheter, einem Führungsdraht, einem Ballon, einem Filter, einem Stent, einem Stentgraft, einem vaskulären Graft, einem vaskulären Patch, einem Shunt und einem intraluminalen Pflastersystem ausgewählt ist, oder
die medizinische Vorrichtung zum Einsetzen in das Gefäßsystem vorbereitet ist.

## Revendications

1. Dispositif médical assurant la libération d'un agent thérapeutique comprenant :
(a) un dispositif médical implantable ou insérable ;
(b) une couche de libération disposée sur au moins une partie du dispositif médical implantable ou insérable, ladite couche de libération comprenant :
(A) un premier polymère qui est un copolymère séquencé ou greffé comprenant (i) une ou plusieurs chaînes de polymère de polyisobutylène et (ii) une ou plusieurs chaînes de polymère styrénique ; et
(B) un second polymère qui est un copolymère séquencé ou greffé comprenant (i) une ou plusieurs chaînes de polymère de polyisobutylène et (ii) une ou plusieurs chaînes choisies parmi l'oxyde de polyéthylène ; le polyvinylpyrrolidone ; le polyacrylamide ; le polydiméthylacrylamide ; l'acide polyacrylique et sels de celui-ci ; l'acide polyméthacrylique et sels de celui-ci ; l'anhydre polymaléique et esters partiels, les acides libres et sels acides de ceux-ci, l'alcool polyvinylique et dérivés complètement et partiellement hydrolysés de celui-ci ; et la polyvinylpyridine,
ou ledit second polymère (B) est un copolymère séquencé ou greffé comprenant (i) une ou plusieurs chaînes de polymères styrénique et (ii) une ou plusieurs chaînes choisies parmi l'oxyde de polyéthylène ; le polyvinylpyrrolidone ; le polyacrylamide ; le polydiméthylacrylamide ; l'acide polyacrylique et sels de celui-ci ; l'acide polyméthacrylique et sels de celui-ci ; l'anhydre polymaléique et esters partiels, les acides libres et sels acides de ceux-ci ; l'alcool polyvinylique et dérivés complètement et partiellement hydrolysés de celui-ci ; et la polyvinylpyridine; et
(C) un agent thérapeutique, dans lequel les phases qui correspondent aux chaînes polymères sont miscibles.

2. Dispositif médical selon la revendication 1,
dans lequel ladite couche de libération est une couche imperméable disposée sur une région contenant l'agent thérapeutique qui comporte ledit agent thérapeutique, ou dans lequel ladite couche de libération est une couche de support comportant ledit agent thérapeutique.

3. Dispositif médical selon la revendication 1,
dans lequel ledit dispositif médical implantable ou insérable est choisi parmi un cathéter, un fil guide, un ballonnet, un filtre, une endoprothèse, une greffe d'endoprothèse, un greffon vasculaire, un patch vasculaire, un shunt et un système de pavage intraluminal.

4. Procédé de formation du dispositif médical de libération d'agent thérapeutique selon la revendication 1, comprenant les étapes suivantes : (a) fournir une solution comprenant : un ou plusieurs solvants, ledit premier polymère (A) et ledit second polymère (B) ; (b) appliquer ladite solution à une surface dudit dispositif médical implantable ou insérable ; et (c) retirer lesdits solvants de ladite solution pour former ladite couche de libération.

5. Procédé selon la revendication 4, dans lequel ladite solution comprend en outre ledit agent thérapeutique.

6. Procédé selon la revendication 4, dans lequel ladite solution est appliquée sur une région contenant l'agent thérapeutique qui comprend ledit agent thérapeutique.

7. Procédé selon la revendication 4, dans lequel ladite solution est appliquée grâce à une technique de pulvérisation de solvant.

8. Utilisation d'un dispositif médical assurant la libération d'un agent thérapeutique selon l'une des revendications 1 à 3 pour la préparation d'un dispositif pour le traitement de la resténose, ou le traitement de maladies et/ou de tumeurs relatives à des organes tels que le colon, le coeur, le système coronarien et vasculaire périphérique, les poumons, la trachée, l'oesophage, le cerveau, le foie, le rein, la vessie, l'urètre et les uretères, l'oeil, les intestins, l'estomac, le pancréas, l'ovaire ou la prostate, ou le traitement de tumeurs relatives à un tissu tel que le muscle squelettique, le muscle lisse, le sein, le cartilage ou l'os, dans lequel le dispositif est préparé pour être implanté ou inséré dans ledit patient.

9. Utilisation selon la revendication 8, dans laquelle
ledit dispositif médical est choisi parmi un cathéter, un fil guide, un ballonnet, un filtre, une endoprothèse, un greffon d'endoprothèse, un greffon vasculaire, un patch vasculaire, un shunt et un système de pavage intraluminal, ou
ledit dispositif médical est préparé pour être inséré dans la vasculature.
